# EUROPEAN PATENT APPLICATION

(11) **EP 1 759 643 A1**
(43) Date of publication of application: **07.03.2007**
(21) Application number: 05425603.7
(22) Date of filing: 30.08.2005
(51) Int. Cl.: A61B 17/17

(54) **Targeting device for intramedullary nails**

(71) Applicant: ORTHOFIX INTERNATIONAL B.V., 1071 Amsterdam (NL)
(72) Inventor: Bardini, Rudj, 46040 Monzambano (Mantova) (IT); Coati, Michele, 37029 San Pietro in Carino (Verona) (IT)
(74) Representative: Botti, Mario

(57) **Abstract**

Targeting device for aligning intra-osseus screws with transverse holes (12a, 12b, 12c, 412a, 412b) of an intramedullary nail (11, 111, 211, 311, 411), of the type comprising an elongated body (13) suitable for being arranged in a predetermined position with respect to the intramedullary nail (11, 111, 211, 311, 411). The device comprises, formed in the elongated body (13), a plurality of guide holes (20a-20n) arranged to be aligned with the transverse holes (12a, 12b, 12c, 412a, 412b) of a multiplicity of intramedullary nails (11) and at least a mask (25, 26, 125a, 125b) suitable for being arranged in a predetermined position on the elongated body (13) to leave selectively accessible amongst the guide holes (20a-20n) only the holes that are suitable for targeting the transverse holes (12a, 12b, 12c, 412a, 412b) of a predetermined intramedullary nail (11, 111, 211, 311, 411) selected from said multiplicity of nails, and to screen the remaining guide holes (20a-20n).

## Description

### Field of application

The present invention refers, in its most general aspect, to the orthopaedic field relating to intramedullary nails.

In its even more particular aspect, the present invention refers to a targeting device for aligning intra-osseus screws with transverse holes of an intramedullary nail.

The targeting device comprises at least one elongated body suitable for being arranged substantially parallel to the intramedullary nails and provided with holes to guide the intra-osseus screws in the transverse holes of the nail.

### Prior art

Targeting devices of the type specified above are known, which allow the fixing screws to be securely targeted in the corresponding transverse holes of a predetermined intramedullary nail inserted in the bone, without requiring the help of X-rays to locate the holes hidden in the bone.

A targeting device is known, for example, from European patent EP 0 772 420 to the Applicant.

The known device, whilst ensuring the desired targeting of the intra-osseus screws in the transverse holes of the intramedullary nail, does nevertheless have recognised drawbacks being not yet solved. The main drawback lies in that, to ensure a correct alignment of the screws, it is necessary to have targeting devices having predetermined guide holes in the elongated body whose position and orientation is strictly linked to the chosen intramedullary nail.

This, although on the one hand ensures a complete correspondence between the transverse holes of the nail and the guide holes of the targeting device, on the other hand however determines a substantial limitation in the possibility of using the same targeting device with a different nail.

Consequently, there is an assortment of targeting devices with guide holes arranged in different positions in the elongated body and it is necessary to select, each time, the one suitable for the intramedullary nail in question.

This drawback is even clearer if one considers that a multiplicity of intramedullary nails are available for a variety of long bones (femur, humerus, tibia) and also, for the same bone, there are multiple modes of application of the nail: anterograde insertion, retrograde insertion, reconstruction mode, pertrochanteric insertion, etc.

Normally, a specific targeting device is suitably constructed for each one of these applications.

The technical problem underlying the present invention is that of providing a targeting device for intramedullary nails that allows all the quoted drawbacks to be overcome, and above all that allows intra-osseus screws to be targeted in a multiplicity of different intramedullary nails and with a limited number of pieces.

### Summary of the invention

Such technical problem is solved by a targeting device of the aforementioned type, which according to the invention comprises, formed in the elongated body, a plurality of guide holes arranged to be aligned with corresponding transverse holes of a multiplicity of intramedullary nails, and at least a mask suitable for being arranged in a predetermined position on the elongated body to leave selectively accessible amongst the guide holes only the holes that are suitable for targeting the transverse holes of a predetermined intramedullary nail selected from said multiplicity and to screen the remaining guide holes of the elongated body.

Preferred embodiments of the targeting device according to the invention are indicated in the dependent claims.

The characteristics and advantages of the targeting device according to the invention will be apparent from the following description of some embodiments, given by way of indicative and non limiting example with reference to the attached drawings.

### Brief description of the drawings

Figure 1 illustrates an axonometric view of the device according to the invention associated with a humeral nail;
Figure 2 illustrates another axonometric view of the device according to the invention;
Figure 3 illustrates a further axonometric view of the device according to the invention with some parts detached;
Figure 4 illustrates a detail of the device of figure 1;
Figure 5 illustrates a further detail of the device according to the invention;
Figure 6 illustrates a view of the device according to the invention with parts detached;
Figures 7a-7c respectively illustrate the device according to the invention in an operative sequence;
Figure 8 illustrates the device according to the invention associated with a femoral nail;
Figure 9 illustrates the device according to the invention associated with a femoral nail for retrograde insertion;
Figure 10 illustrates the device according to the invention associated with a pertrochanteric femoral nail;
Figures 11 and 12 respectively illustrate the device according to the invention, associated with a tibial nail in two different angular positions;
Figure 13 illustrates the device according to the invention in accordance with a further embodiment and associated with a humeral nail;
Figure 14 illustrates the device of figure 13 associated with a femoral nail.

### Detailed description

With reference to the attached figures, a targeting device for aligning intra-osseus screws (not illustrated in the drawings) with transverse holes of an intramedullary nail is globally indicated with reference number 10.

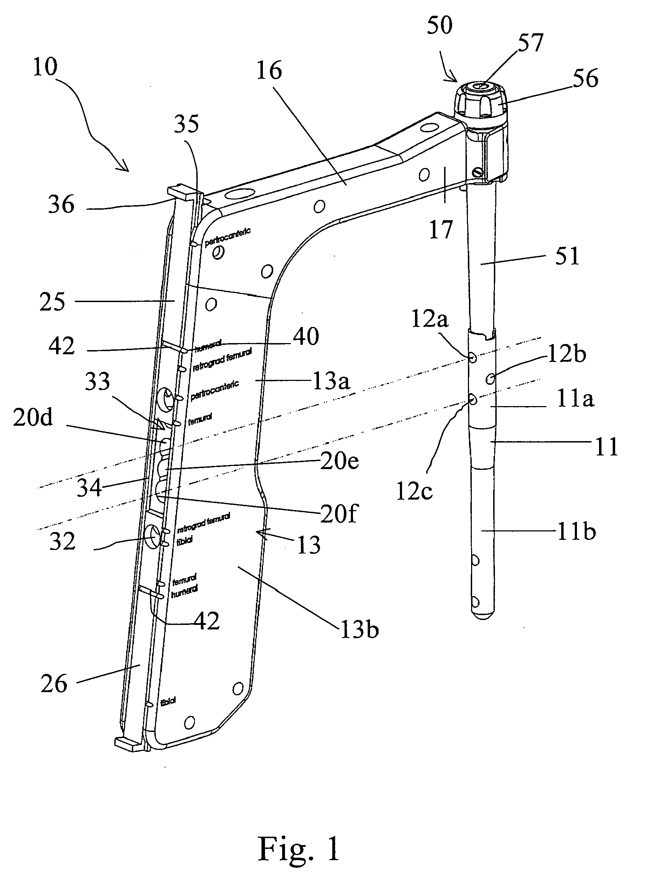

In particular, in the solution illustrated in figures 1 to 7c, the application of the targeting device 10 for an intramedullary nail 11 for a humerus having a proximal portion 11a and a distal portion 11b is described. The device 10 is intended to target the transverse holes 12a, 12b, 12c of the proximal portion 11a.

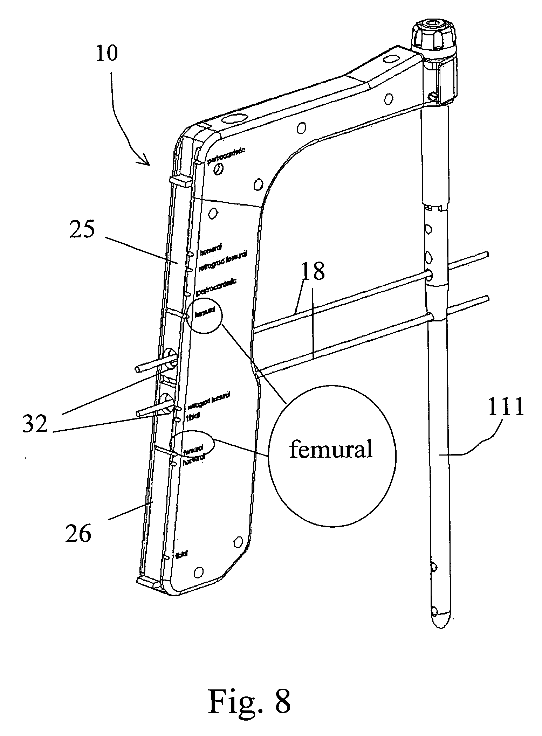

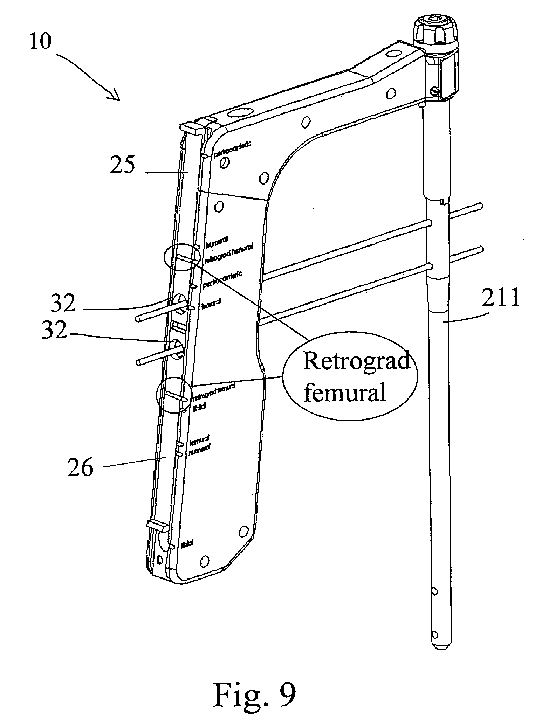

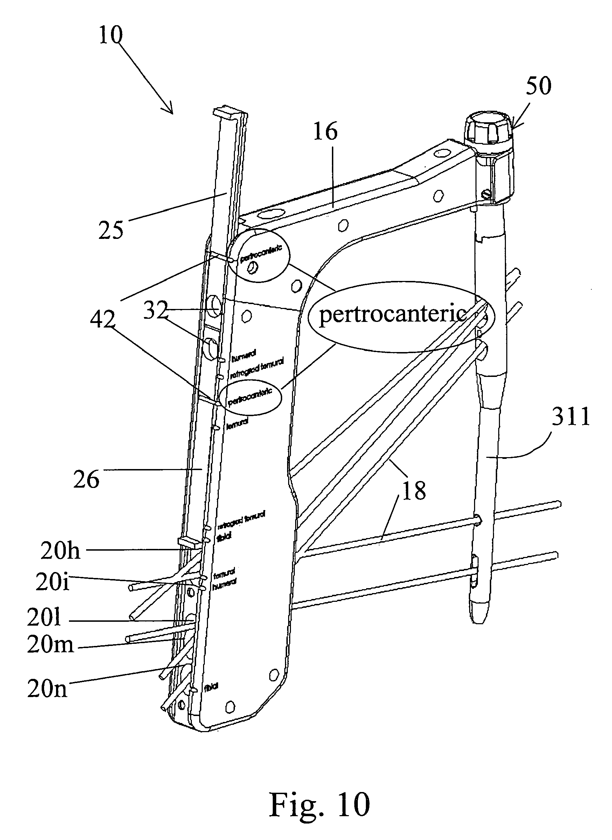

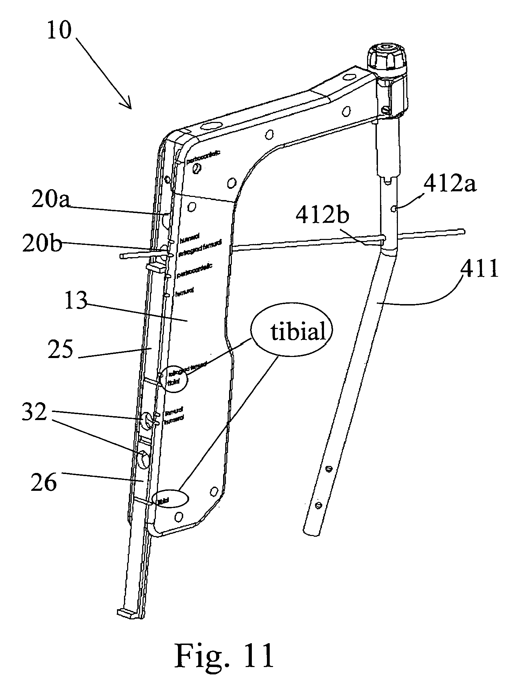

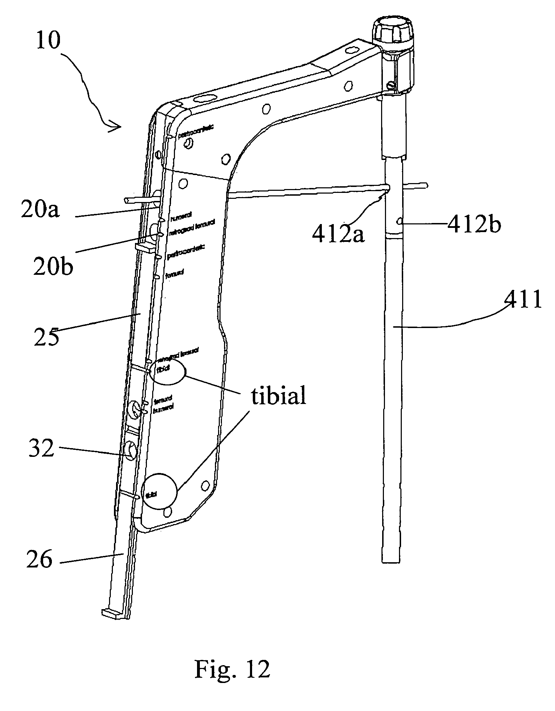

In the solutions of figures 8, 9, 10, 11 and 12, the targeting device 10 is illustrated applied respectively to a multiplicity of different nails: a femoral nail 11 (figure 8), to a femoral nail 211 intended for retrograde insertion (figure 9), to a pertrochanteric femoral nail 311 (figure 10), and to a tibial nail 411 (figures 11 and 12).

By way of illustration, the following description will mainly refer to the targeting device 10 for aligning intra-osseus screws with the transverse holes of the humeral nail 11, it being understood that the same description is valid for the application of the targeting device 10 to the remaining intramedullary nails.

Moreover, the following description, purely as an example, will refer to the use of the targeting device 10 for the proximal portion 11a of the nail 11, even though there is no reason why the device 10 cannot be arranged to target the holes of the distal portion 11b or of both the portions.

In particular, as it can be observed in figure 1, the two most external transverse holes 12a and 12c are aligned to each other and lie on the same plane whereas the intermediate transverse hole 12b is arranged on a plane forming an angle, for example of 90°, with the plane of the other two holes 12a, 12c.

The device 10 comprises an elongated body 13 and a handle 16.

The body 13 is handle-shaft-like shaped and is suitable for being arranged in a predetermined position with respect to the intramedullary nail 11 substantially parallel to this latter, on the same plane wheron the intramedullary nail 11 itself lies.

The handle 16 connects the elongated body 13 to the nail 11 and it is arranged substantially perpendicular to the nail 11 and to the elongated body 13 so as to form a parallelogram-shaped structure with them, which allows a predetermined spaced relationship of the body 13 with respect to the nail 11 to be obtained.

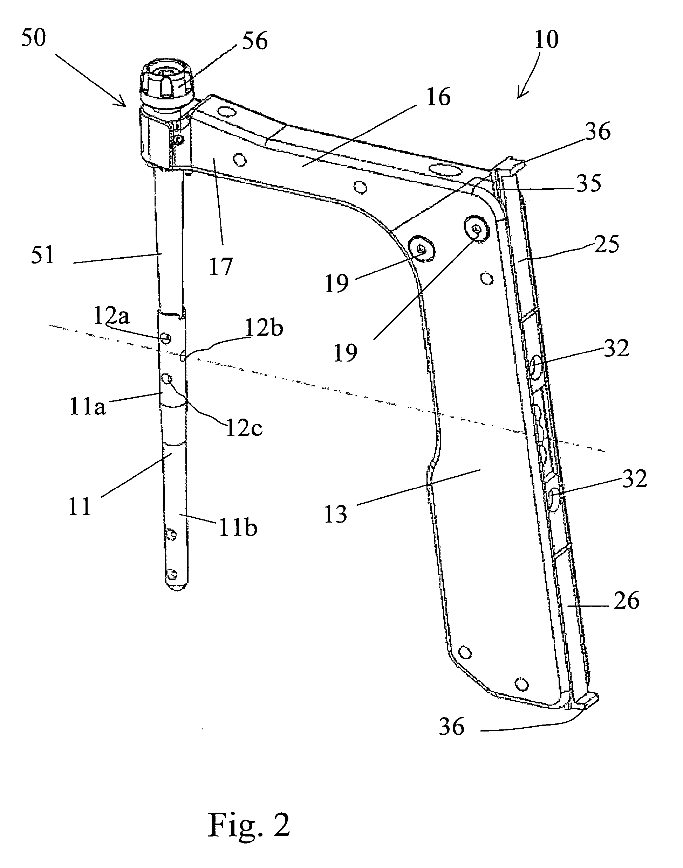

The handle 16 is fixed in a known way to the body 13 through screws 19 (figure 2).

Further characteristics on the handle 16 and the modes of connection thereof to the nail 11 shall be described in greater detail hereafter in the description.

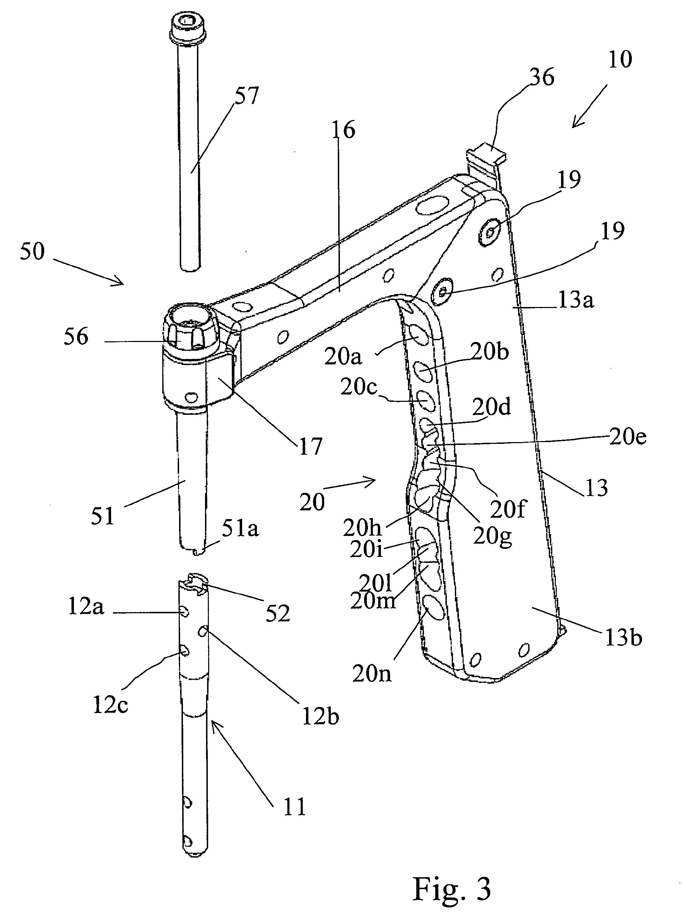

The targeting device 10 comprises, formed in the body 13, a plurality of guide holes generally indicated in figure 3 with reference number 20, arranged aligned with the corresponding transverse holes of the multiplicity of intramedullary nails, i.e. of the aforementioned intramedullary nails intended for different long bones, such as the femur, the tibia, the humerus and, within the same bone, for different applications, such as anterograde insertion mode, retrograde insertion mode, reconstruction mode, etc.

In particular, with reference to figure 3, in the illustrated device, the guide element 13 comprises two holes 20a and 20b for the alignment of corresponding intra-osseus screws in the tibial nail 411 (figures 11 and 12), two holes 20c and 20e for the femoral nail 211 with retrograde insertion (figure 9), three holes 20d, 20e and 20f for the humeral nail 11 (figures 1 and 2), two holes 20e and 20g for the femoral nail 111 (figure 8), and five holes 20h, 20i, 201, 20m and 20n for targeting holes of the pertrochanteric femoral nail 311 (figure 10).

As it can be seen, some of the nails quoted above have common guide holes on the body 13.

The alignment of the guide holes with the respective transverse holes of the nails is indicated in figures 8-12 through alignment shafts 18.

According to the invention, the device 10 comprises at least one mask suitable for being arranged in a predetermined position on the elongated body 13 to leave selectively accessible, among the plurality of guide holes 20, only the holes that are suitable for targeting the transverse holes of a predetermined intramedullary nail selected from said multiplicity, and screening the remaining guide holes 20 of the body 13.

In other words, the device 10 comprises an element, preferably structurally independent from the body 13, which can be associated with the latter to screen the guide holes that are not used for a given application.

In the solution illustrated in figures 1-12, the mask is divided into at least two identical mask portions 25 and 26, each of which is intended to screen and leave selectively accessible the guide holes 20a-20n of a portion of the body 13.

In particular, the mask portion 25 is intended to selectively screen the guide holes of a proximal portion 13a of the body 13, whereas the mask portion 26 is intended to selectively screen the holes of a distal portion 13b of the body 13.

The guide holes 20a-20n are distributed in such a way that, by moving each mask portion 25 and 26 into predetermined positions on the elongated body 13, it is possible to leave the guide holes 20a-20n selectively accessible according to the intramedullary nail used, and to screen the others.

The use of tow mask portions 25, 26 allows a plurality of combinations of screened holes and accessible holes for a large number of intramedullary nails to be systematically obtained so as to obtain a very versatile targeting device.

In particular, in figures 1 and 2 the mask portions 25 and 26 are arranged so as to leave accessible the three central guide holes 20d, 20e and 20f that, as stated above, can be aligned with the transverse holes 12a, 12b, 12c of the proximal portion 11a of the humeral nail 11.

With reference to figure 8, the two mask portions 25 and 26 are arranged so as to leave free the two guide holes 20e and 20g for the femoral nail 11; with reference to figure 9, the two mask portions 25 and 26 are arranged so as to leave free the two holes 20c and 20e for the femoral nail 211 with retrograde insertion; with reference to figure 10, the two mask portions 25 and 26 are arranged so as to leave accessible the five holes 20h, 20i, 201, 20m and 20n for the pertrochanteric femoral nail 311; with reference to figures 11 and 12, the two mask portions 25 and 26 are arranged so as to leave accessible two guide holes 20a and 20b for the alignment of corresponding intra-osseus screws in the tibial nail 411.

It can be seen that, thanks to the use of the mask portions 25, 26, only the relevant guide holes for the nail used are accessible to the surgeon, and thus the surgeon does not run the risk of making incorrect perforations.

Preferably, the movement of the mask portions 25 and 26 from one position to the other according to the nail used is obtained through the sliding of the same. The mask portions 25, 26 are mobile along a wall of the body 13 on the opposite side to the one facing the nail 11.

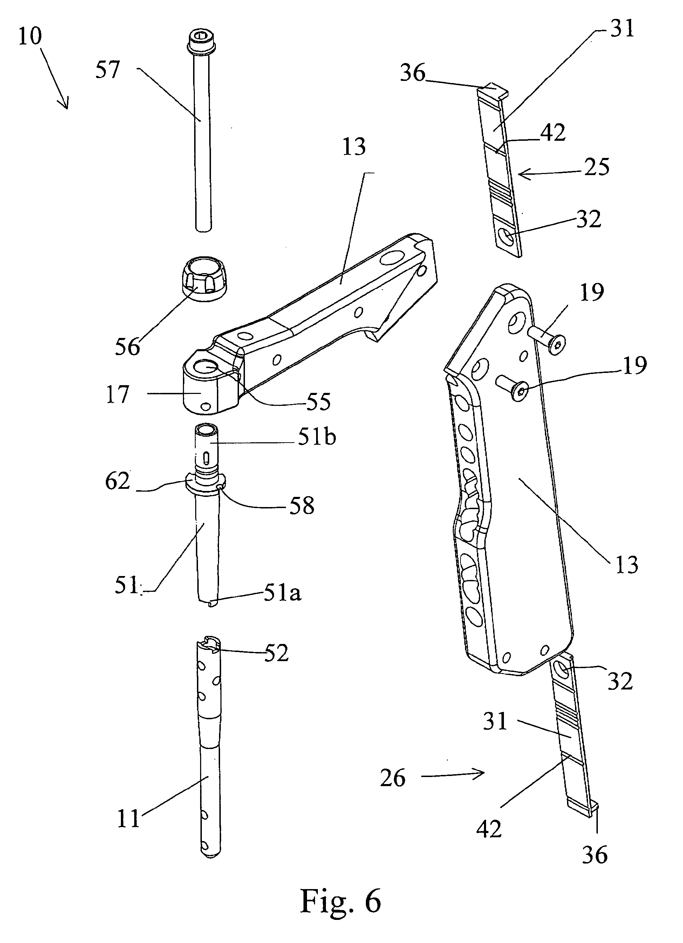

Regarding this, each mask portion 25 and 26 consists of a substantially rectangular blade with the function of slider 31, or cursor, (figure 6) wherein a through hole 32 is formed.

It should be noted that the through hole 32 has the function of leaving accessible the guide holes 20 of the body 13 when the mask portions 25, 26 are arranged in predetermined positions for a certain application. For example, with reference to figure 8, the mask portions 25 and 26 are arranged so that the through holes 32 are aligned with the respective guide holes 20e and 20g for the femoral nail 111; whereas in the case of figure 9, the through holes 32 are aligned with the respective guide holes 20c and 20e for the retrograde femoral nail 211.

In these last cases, the through hole 32 is arranged in such a way as to perfectly coincide with the corresponding guide hole to be left accessible.

It should be noted that, in the case of the solution of figures 1 and 2, the holes 20d, 20e, 20f are left directly accessible by the masks 25, 26 and in this case the through hole 32 has no function. Similarly, in the case of application of the targeting device 10 in association with the pertrochanteric femoral nail 311 (figure 10), and with the tibial nail 411 (figures 11 and 12), the through holes 32 have no function. The relative guide holes are left accessible by the mask portions 25 and 26.

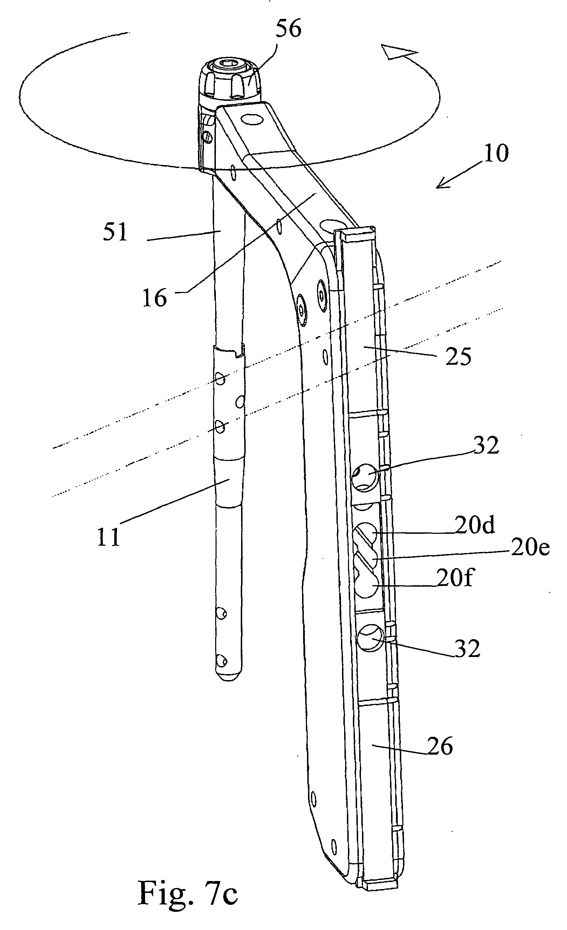

Indeed, it should be noted that, in these last cases (see figures 7c, 10, 11 and 12), the through holes 32, having no function, do not precisely coincide with any guide hole 20 but they partially overlap the underlying holes, so as to screen them and not allow the surgeon to commit a mistake.

To allow the sliding of the masks 25 and 26, the device 10 comprises a seat 33 that extends passing from one end to the other of the targeting body 13.

The seat 33 is provided with two longitudinal ribs 34 which slidably insert themselves into corresponding opposite rails 35 formed on both sides of the respective mask portions 25, 26 and that allow a back and forth movement of these latter.

Each mask portion 25 and 26 is completed by a projection 36 that makes it easier to be held.

In order to make it easier to identify the correct position of each mask portion 25 and 26 on the body 13, the device 10 is completed by a plurality of reference notches 40 formed on an edge of the seat 33 of the body 13, in the proximal portion 13a and in the distal portion 13b of the body 13, respectively.

The mask portions 25, 26 are provided with corresponding notches 42 formed on its exposed face so as to be clearly visible. In the illustrated solution the notches 42 are formed on both faces of the mask portions 25 and 26. By selectively aligning each notch 42 of each mask portion 25 and 26 with one or other reference notch 40, based upon the selected intramedullary nail, it is possible to identify the correct position of the mask portions 25, 26 to be set.

It should also be noted that each of the reference notches 40 is indicated with the name of the selected nail or else the application mode of a given nail. In the solution illustrated in the figures, the following terms are indicated: tibial, humeral, femoral, retrograde femoral, pertrochanteric.

The terms are in pairs, to allow the positioning of both of the mask portions 25 and 26 for the same application.

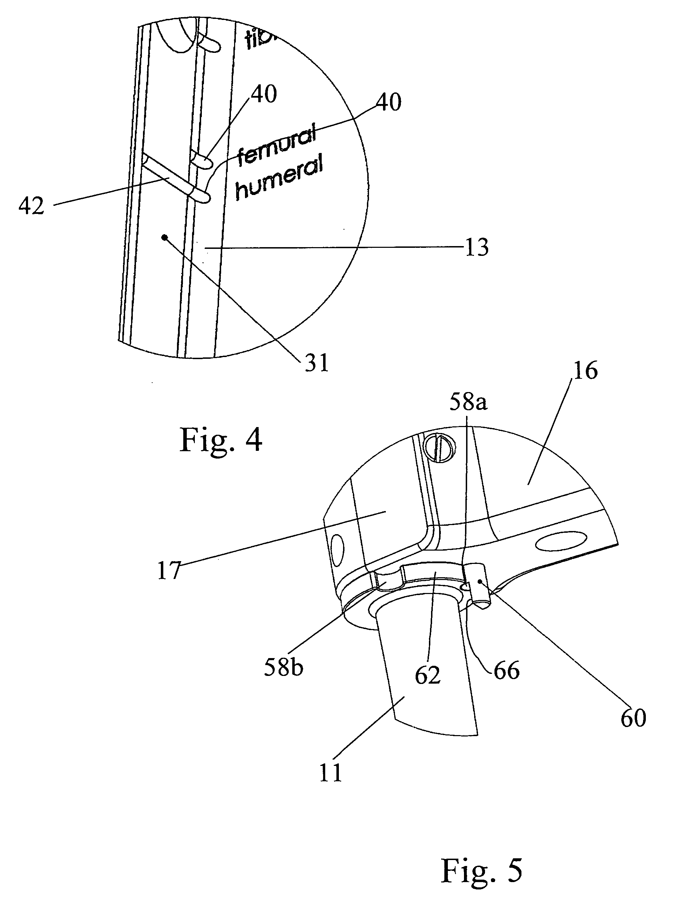

Figure 4 illustrates in detail the alignment of the respective notches 40 and 42 of the body 13 and of the masks 25 and 26 in the case of application of the aforementioned humeral nail 11, and similarly figure 8 illustrates the alignment of the notches 40, 42 for the femoral nail 111, figure 9 for the femoral nail with retrograde insertion, figure 10 for the pertrochanteric nail 311 and figures 11 and 12 for the tibial nail 411.

With reference to figures 3, 5, 6, 7a-7c another characteristic of the targeting device according to the present invention shall now be illustrated.

In particular, this concerns a structure 50 for the connection of the handle 16 to the nail 11.

In particular, the connection structure 50 is shaped to angularly orientate the handle 16 and the body 13 with respect to the nail 11, so as to angularly move the body 13 around the nail 11 and allow all the transverse holes 12a, 12b, 12c of the nail 11 to be targeted which, as stated above, are arranged on planes forming an angle with each other.

The structure 50 is also shaped to move the body 13 without disconnecting the targeting device 10 from the nail 11.

In particular, the structure 50 comprises an attachment element 51 which is in the form of a substantially cylindrical sleeve that is arranged in axial extension on the head of the nail 11.

The attachment element 51 comprises a pair of opposite projections 51a intended to engage a cut-like slot 52 for screwdriver formed on the head of the nail 11 and defining a positioning line having a predetermined fixed angular relationship with respect to the axis of each transverse hole 12a, 12b, 12c of the nail 11.

In the case of the illustrated solution the slot 52 defines a line lying on a plane perpendicular to the plane of the holes 12a, 12c and lying on the same plane as the intermediate transverse hole 12b.

It should also be noted that the attachment element 51 comprises a head portion 51b limited at the bottom by a collar 62 that is formed integrally on the outer surface of the attachment element 51.

The head portion 51b is inserted with clearance in a suitable cylindrical seat 55 formed in an end portion 17 of the handle 16 that is substantially tubular shaped.

The structure 50 also comprises a lock nut 56, which is screwed onto the head portion 51 b of the attachment element 51 to stably lock it in the seat 55 of the handle 16 in a predetermined angular position.

The device 10 also comprises a screw 57 with the function of a tightening element, which is inserted in the attachment element 51 and screwed into the head of the nail 11 to tighten the handle 16 to the nail 11.

As stated above, the attachment element 51 and the seat 55 are suitably shaped with an intermediate clearance to control the angular movement of the attachment element 51, and therefore of the intramedullary nail 11, with respect to the handle 16 according to predetermined angles.

For such purpose, the attachment element 51 comprises a plurality of recesses 58a, 58b distributed radially on the collar 62 (figure 5) and the handle 16 comprises a fastening pin 60 arranged below the portion 17 next to the attachment element 51. The fastening pin 60 is intended to be selectively engaged in one of the recesses 58a, 58b. In the illustrated solution two recesses 58a and 58b are provided forming an angle of 90° with each other. The recess 58a is aligned on the same plane as the projections 51a of the attachment element 51.

To allow the angular movement of the elongated body 13 with respect to the nail 11, the targeting device 10 is actuated in the following way.

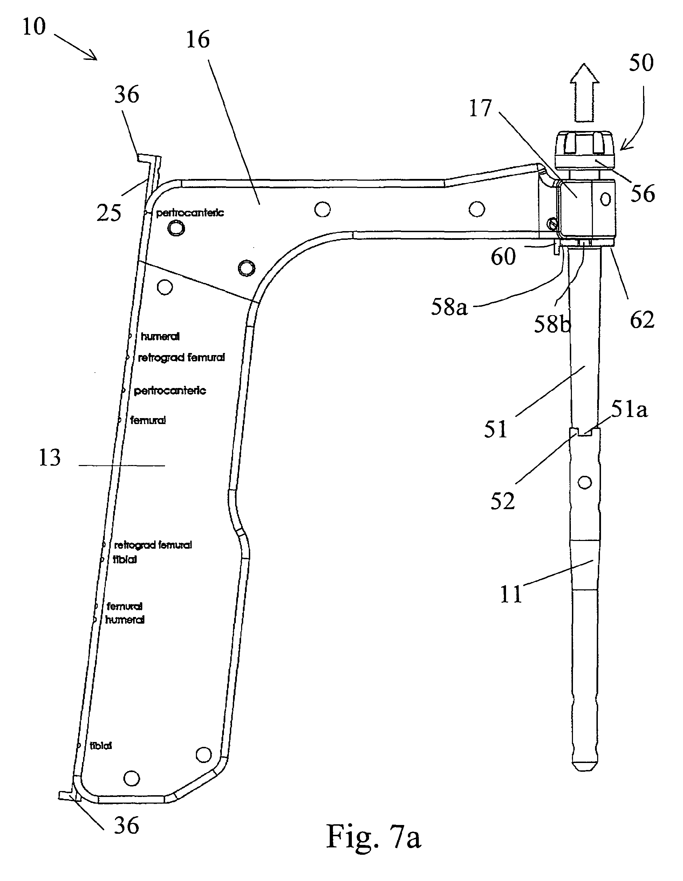

Initially, after having connected the attachment element 51 to the nail 11, the handle 16 is mounted on the attachment element 51 and fixed through the screw 57 and the nut 56. As it can be seen in figures 1 and 7a, by locking the nut 56, the fastening pin 60 is initially inserted into the recess 58a so that the handle 16 is arranged substantially perpendicular to the line defined by the slot 52 and by the projections 51a of the attachment element 51. The alignment of the guide holes 20d and 20f with the transverse holes 12a and 12c of the nail 11 is thus ensured (figure 1).

In such position it is now possible for the surgeon to carry out the necessary operations, like for example the drilling of the bone and the screwing of the intra-osseus screws into the two transverse holes 12a and 12c.

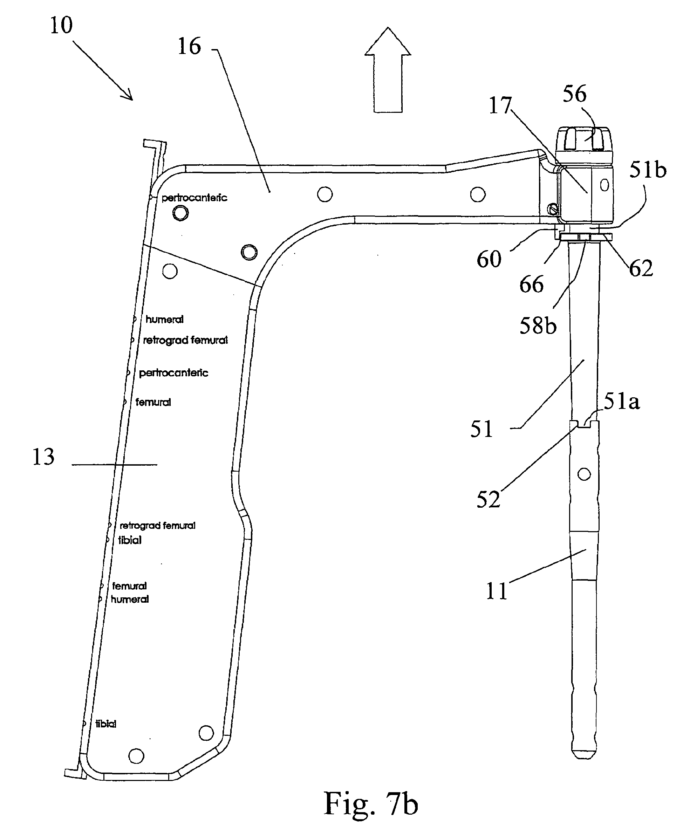

Then, to carry out the angular movement, the lock nut 56 and the screw 57 are unscrewed, and the lock nut 56 is lifted from the head of the attachment element 51 (figure 7a), so as to allow an upward movement in the axial direction of the handle 16 with respect to the attachment element 51 (figure 7b). The latter thus slides axially, thanks to the aforementioned clearance, in the seat 55.

Such movement allows a disengagement of the fastening pin 60 from the relative recess 58a.

Once the disengagement has been obtained it is possible to rotate the handle 16 (figure 7c) with respect to the intramedullary nail 11, moving the fastening pin 60 around the collar 62 of the attachment element 51 until the next recess 58b is reached at a desired angular position, like for example that of figure 2 moved by 90° in which the guide hole 20e is aligned with the transverse hole 12b of the nail 11.

It should be noted that (figure 5) the fastening pin 60 is provided with an end turning 66, which reduces the bulk of the end portion of the fastening pin 60 and allows the angular movement thereof from one recess 58a towards the next 58b around the collar 62, when the handle 16 is lifted with respect to the attachment element 51.

Once the desired angular position has been reached, wherein the pin 60 is arranged at the predetermined recess 58b, the handle 16 is lowered again and the nut 56 and the screw 57 are again screwed together to obtain the condition illustrated in figure 2.

It can be seen that, when carrying out all these movements, the attachment element 51 remains in the seat 55 and, at the same time, the projections 51a of the attachment element 51 are never disconnected from the nail 11, thus allowing a constant connection of the entire targeting device 10 to the nail 11.

Once the new desired angular position has been obtained it is now possible for the surgeon, according to the predetermined nail, and according to the angular position with respect to the nail, to carry out the necessary operations, like for example the drilling of the bone and the screwing of the intra-osseus screws.

The operation described above to angularly move the targeting device 10 around the nail is used in a similar way to target the proximal holes 412a and 412b of the tibial nail 411 of figures 11 and 12 respectively, which, as for the humeral nail, are on planes forming an angle with each other.

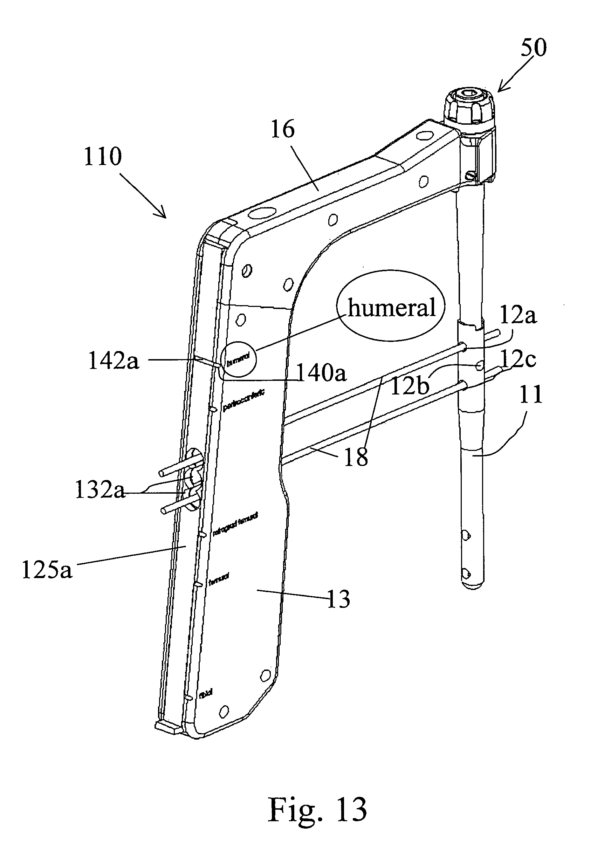

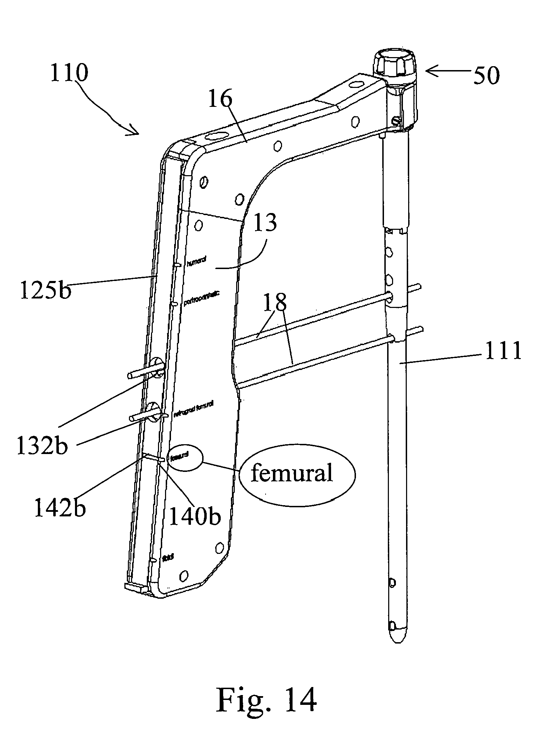

With reference to figures 13 and 14, a further embodiment of the targeting device according to the invention shall now be illustrated, wherein the targeting device 10 comprises a plurality of masks 125a, 125b, one for each nail of the multiplicity of nails. Basically, unlike the previous solution wherein both the mask portions 25, 26 systematically satisfy a wide range of combinations of accessible holes and screened holes, in this solution the use is provided of a plurality of masks, each of which is intended to screen and make unequivocally accessible only the guide holes for a selected nail.

By way of illustration, the parts of the device having the same function and structure already described previously for the first embodiment of the invention keep the same reference numbers.

Figure 13, for example, illustrates a mask 125a for the humeral nail 11, whereas figure 14 illustrates a mask 125b for the femoral nail 111.

Each mask 125a, 125b is inserted through sliding in the relative seat 33 of the body 13 and extends along the entire body 13.

Each mask 125a, 126b is provided with respective holes 132a, 132b intended to be unequivocally aligned with the guide holes that must be made accessible for the corresponding selected nail.

In this case, to make it easier for the surgeon to select the correct mask 125a, 125b for a given application, each mask 125a, 125b is provided with means for indicating the application for which the mask itself is intended, for example humeral, femoral, tibial application and similar.

In the case of the illustrated solution, each mask 125a, 125b is provided with a notch 142a, 142b which, according to the predetermined nail, is intended to be arranged at a predetermined reference notch 140a, 140b formed on the body 13 on a side of the seat 33. The reference notch 14a, 140b is identified by a wording indicating the name of a predetermined nail.

The correspondence between the notch 142a and the reference notch 140a of figure 13 ensures the surgeon that it is the correct mask 125a for the humeral nail 11.

Similarly, the correspondence between the notch 142b and the reference notch 140b of figure 14 ensures the surgeon that it is the correct mask 125b for the femoral nail 111.

The main advantage of the present invention lies in the fact that a single targeting device is used to target intra-osseus screws for multiple intramedullary nails, and for different applications in the same bone, also allowing a surgeon to easily and quickly identify the holes to be used.

In fact, regarding this it should be highlighted that the correct position of the two mask portions, or else the selection of the correct mask from the plurality of masks in accordance with the further embodiment, it is preset prior to the operation, thus allowing the surgeon in the operating theatre to immediately see the guide holes to be used.

Another advantage of the present invention lies in the fact that it is possible to angularly move the targeting device without removing the connection with the nail, thus allowing the same targeting device to be used to insert intra-osseus screws on planes forming angles with each other around the axis of the nail.

This allows a surgeon to insert the screws quickly without having to replace the targeting device, or having to disconnect it each time from the nail.

A further advantage of the present invention lies in the small number of components of the device; for example, with the two mask portions described above it is possible, as stated above, to satisfy a large number of screened holes/accessible holes combinations thus allowing the use of the same targeting device for multiple intramedullary nails.

Of course, a man skilled in the art can make numerous modifications and variants to the targeting device described above in order to satisfy contingent and specific requirements, all of which are in any case covered by the scope of protection of the invention as defined by the following claims.

## Claims

1. Targeting device for aligning intra-osseus screws with transverse holes (12a, 12b, 12c, 412a, 412b) of an intramedullary nail (11, 111, 211, 311, 411), of the type comprising at least one elongated body (13) suitable for being arranged substantially parallel to the intramedullary nail (11, 111, 211, 311, 411), **characterised in that** it comprises, formed in the elongated body (13), a plurality of guide holes (20a-20n) arranged to be aligned with the transverse holes (12a, 12b, 12c, 412a, 412b) of a multiplicity of intramedullary nails (11) and at least a mask (25, 26, 125a, 125b) suitable for being arranged in a predetermined position on the elongated body (13) to leave selectively accessible amongst the guide holes (20a-20n) only the holes that are suitable for targeting the transverse holes (12a, 12b, 12c, 412a, 412b) of a predetermined intramedullary nail (11, 111, 211, 311, 411) selected from said multiplicity of nails, and to screen the remaining guide holes (20a-20n).

2. Device according to claim 1, **characterised in that** the mask can be slidably moved in a relative seat (33) formed on the elongated body (13) to be selectively arranged in said predetermined position.

3. Device according to claim 1, **characterised in that** said mask (25, 26) comprises a substantially rectangular blade wherein a through hole (32, 132a, 132b) is formed intended to coincide in said predetermined position with a guide hole (20) of the body (13) to allow it to be accessed.

4. Device according to claim 1, **characterised in that** the mask is divided into at least two mask portions (25, 26) intended to be arranged in a predetermined portion (13a, 13b) selected from a plurality of positions according to the selected nail.

5. Device according to claim 4, **characterised in that** the two mask portions (25, 26) are identical to each other.

6. Device according to claim 4, **characterised in that** it comprises indication means (40, 41) for indicating the predetermined position of the mask (35, 36) on the body (13).

7. Device according to any one of the previous claims 1 to 3, **characterised in that** it comprises a plurality of masks (125a, 125b) each intended to screen and make unequivocally accessible only the guide holes of a certain nail (11, 411) selected from said multiplicity of intramedullary nails (11, 111, 211, 311, 411).

8. Device according to claim 7, **characterised in that** each mask is provided with indication means (140a, 140b, 142a, 142b) to indicate the intramedullary nail for which it is intended.

9. Device according to claim 6 or 8, **characterised in that** said indication means comprise at least one notch (42, 142a, 142b) formed on said mask (25, 26, 125a, 125b) suitable for being aligned with a predetermined reference notch (40, 140a, 140b) made on the elongated body (13).

10. Device according to claim 9, **characterised in that** said reference notch (40, 140a, 140b) is identified through a wording indicating the nail for which a predetermined mask (25, 26, 125a, 125b) is intended.

11. Device according to claim 9, **characterised in that** it comprises a plurality of reference notches (40, 140a, 140b) formed on a side of the elongated body (13).

12. Device according to claim 1, **characterised in that** it comprises a handle (16) integral with the elongated body (13) to connect the elongated body (13) with the nail (11) and means for angularly orientating the elongated body (13) and the handle (16) around the nail (11) in predetermined angular positions.

13. Device according to claim 12, **characterised in that** it comprises an attachment element (51) suitable for being arranged in axial extension of the nail (11) and which comprises a portion (51b) inserted with clearance in a suitable seat (55) formed in an end portion (17) of the handle (16) to allow an axial movement of the handle (16) with respect to the attachment element (51) and a subsequent rotation of the handle (16) together with the elongated body (13), according to predetermined angles with respect to the nail (11).

14. Device according to claim 13, **characterised in that** to control the angular movement, the attachment element (51) comprises an outer circular collar (62) and a plurality of recesses (58a, 58b) distributed radially on the circular collar (62) and the handle (16) comprises a fastening pin (60) arranged next to the attachment element (51) and intended to turn around the collar (62) to be selectively engaged in one of the recesses (58a, 58b).

15. Device according to claim 14, **characterised in that** said fastening pin (60) comprises an end turning (66) to allow the rotation of the fastening pin (60) around the collar (62) between one recess (58a) and a subsequent recess (58b).

16. Device according to claim 13, **characterised in that** it comprises a lock nut (56), which is suitable for being screwed into the attachment element (51) to stably lock it into the seat (55) of the handle (16) in a predetermined angular position with respect to the elongated body (13).

17. Device according to claim 16, **characterised in that** it comprises a screw (57) with the function of a tightening element, which is inserted into the attachment element (51) and screwed into the head of the nail (11) to tighten the handle (16) to the nail (11).
